# EUROPÄISCHE PATENTSCHRIFT

(11) **EP 2 902 327 B1**
(45) Veröffentlichungstag und Bekanntmachung des Hinweises auf die Patenterteilung: **27.01.2016**
(21) Anmeldenummer: 14000375.7
(22) Anmeldetag: 01.02.2014
(51) Int. Cl.: B65B 1/36, B65B 1/04, A61M 15/00

(54) **Doseiereinrichtung für Pulver und Verfahren zur Dosierung von Pulver**
Metering device for powder and method for metering of powder
Dispositif de dosage pour poudre et procédé de dosage de poudre

(43) Veröffentlichungstag der Anmeldung: 05.08.2015
(73) Patentinhaber: Harro Höfliger Verpackungsmaschinen GmbH, 71573 Allmersbach im Tal (DE)
(72) Erfinder: Wolf, Achim, 71573 Allmersbach im Tal (DE)
(74) Vertreter: Zurhorst, Stefan

(56) Entgegenhaltungen:
- EP-A2- 0 022 541
- WO-A1-97/41031
- DE-A1-102010 000 682
- US-A- 3 718 164

## Beschreibung

Die Erfindung betrifft eine Dosiereinrichtung zum volumetrischen Dosieren eines insbesondere pharmazeutischen Pulvers der im Oberbegriff des Anspruchs 1 angegebenen Gattung, ein Dosiersystem sowie ein Verfahren zur Dosierung eines solchen Pulvers mittels der genannten Dosiereinrichtung und zur gleichzeitigen Befiillung von mehreren in einer gemeinsamen Ebene angeordneten Zielkavitäten.

Für die ordnungsgemäße Verwendung von beispielsweise pharmazeutischem Pulver durch die Zielperson müssen exakt dosierte Teilmengen beispielsweise in Kapseln, Blisterverpackungen oder anderen Zielkavitäten bereitgestellt werden. Verbreitet findet hierfür zunächst eine volumetrische Dosierung des Pulvers statt. Die dosierten Teilmengen werden dann in die Zielkavitäten eingefüllt.

Sofern die Zielkavitäten in einer Reihe angeordnet sind, werden verbreitet sogenannte Walzendosierer eingesetzt. Hierbei sind auf der Mantelfläche einer Dosierwalze Dosieröffnungen entlang einer Linie parallel zur Walzenlängsachse vorgesehen, die mit Pulver befüllt werden. Das Volumen der Dosieröffnungen definiert einzelne Teilmengen des Pulvers, wodurch eine volumetrische Dosierung vorgenommen wird. Die lineare Reihe von Dosieröffnungen korrespondiert zur linearen Reihe von Zielkavitäten. Nach Befüllung der Dosieröffnungen wird die Dosierwalze soweit gedreht, dass alle Dosieröffnungen über den Zielkavitäten liegen. Das volumetrisch dosierte Pulver wird dann aus den Dosieröffnungen in die Zielkavitäten überfährt.

Problematisch werden Dosierung und Befüllung insbesondere dann, wenn eine größere Anzahl von Zielkavitäten, welche nicht in einer Linie, sondern in einer gemeinsamen Ebene angeordnet sind, gleichzeitig befüllt werden sollen. Dieser Fall tritt zum Beispiel bei sogenannten DPI-Discs (Dry Powder Inhaler Disc) auf, bei denen mehrere, insbesondere 10 bis 61 Zielkavitäten kreisförmig in einer ebenen Trägerscheibe ausgebildet und von einer Siegelfläche umgeben sind. Alle Zielkavitäten einer solchen Ebene sollen gleichzeitig befüllt und anschließend durch Aufsiegelung einer Siegelfolie auf die Siegelfläche verschlossen werden. In solchen oder vergleichbaren Fällen scheidet die Befüllung mit Walzendosierern aus, da die Dosieröffnungen nur auf einer achsparallelen Linie der Walzenmantelfläche und nicht in einer Ebene angeordnet sein können. Es wäre deshalb nur möglich, zwei diametral sich gegenüberliegende Zielkavitäten gleichzeitig zu befüllen. Für die vollständige Befüllung aller in einer Ebene liegenden Zielkavitäten wären also bei Verwendung eines Walzendosierers mehrere sequentielle Füllvorgänge erforderlich, was unwirtschaftlich ist.

Ein praktikabler Weg ist die Befüllung mit sogenannten Stechhebern, wenngleich eine entsprechend hohe Anzahl von einzelnen Stechhebern mit entsprechend hohem Investitionsaufwand erforderlich ist. Der Einsatz von Stechhebern ist in solchen Fällen technisch problematisch, bei denen die Zielkavitäten einen unrunden Grundriss aufweisen. Außerdem muss ein hinreichend tiefes Pulverbett bereitgestellt werden, in dessen Folge sich eine unerwünscht große Pulverrestmenge nicht vermeiden lässt. Alternativ können sogenannte Membrandosierer eingesetzt werden, bei denen die Zielkavitäten mit einer luftdurchlässigen Membran abgedeckt werden. Durch die Membran ist ein Pulverkanal hindurchgeführt. Durch Aufbringung einer negativen Druckdifferenz an der Membran wird Pulver durch den Pulverkanal in die Zielkavität eingesaugt und an der Membran zurückgehalten. Vorrichtung und Verfahren sind darauf beschränkt, eine randvolle Befüllung der Zielkavität herbeizuführen. Die Verbindung von Membran und Pulverkanal ist technisch problematisch, wobei außerdem die wirksame Membranfläche durch den Querschnitt des hindurch geführten Pulverkanals reduziert wird. Der Querschnitt des Pulverkanals muss deshalb so klein wie möglich gewählt werden, was aber einem zuverlässigen Durchfluss des Pulvers entgegensteht. Auch hier können Probleme bei der Befüllung von im Grundriss unrunden Zielkavitäten auftreten, sofern in schmalen Grundrissbereichen kein ausreichend großer Platz für die Membran abseits des Pulverkanals verbleibt.

US-A-3 718 164 zeigt eine Dosiereinrichtung mit einem Pulvervorrats behälter mit Dosieröffnungen in denen Pulver durch zwei Schaber abgemessen wird.

Der Erfindung liegt die Aufgabe zugrunde, eine Dosiereinrichtung zum volumetrischen Dosieren eines insbesondere pharmazeutischen Pulvers anzugeben, welche verbesserte Möglichkeiten zur gleichzeitigen Befüllung von mehreren in einer gemeinsamen Ebene angeordneten Zielkavitäten eröffnet.

Diese Aufgabe wird durch eine Dosiereinrichtung mit den Merkmalen des Anspruchs 1 gelöst.

Der Erfindung liegt noch die Aufgabe zugrunde, ein Dosiersystem umfassend eine Anzahl von in einer gemeinsamen Ebene angeordneten Zielkavitäten und eine geometrisch darauf abgestimmte Dosiereinrichtung anzugeben, mittels dessen die Zielkavitäten gleichzeitig befüllt werden können.

Diese Aufgabe wird durch ein Dosiersystem mit den Merkmalen des Anspruchs 9 gelöst.

Der Erfindung liegt des Weiteren die Aufgabe zugrunde, ein Dosier- und Füllverfahren anzugeben, mittels dessen mit erhöhter Genauigkeit und Zuverlässigkeit bei hohen Taktraten Einzelmengen von Pulver dosiert und gleichzeitig in mehrere in einer gemeinsamen Ebene angeordnete Zielkavitäten eingefüllt werden können.

Diese Aufgabe wird durch ein Verfahren mit den Merkmalen des Anspruchs 10 gelöst.

Nach der Erfindung ist vorgesehen, dass die Dosiereinrichtung eine Dosierstation mit einem Pulvervorratsbehälter, eine Füllstation und ein bewegliches Dosierorgan umfasst, wobei das Dosierorgan zyklisch von der Dosierstation zur Füllstation und wieder zurück verfahrbar ist. Das Dosierorgan weist eine ebene Transferfläche auf, in der korrespondierend zur Anzahl und geometrischen Verteilung der Zielkavitäten mehrere Dosierkavitäten eingearbeitet sind. Die Dosierstation weist eine ebene Anlagefläche auf, die zur dichtenden Anlage des Dosierorgans mittels seiner Transferfläche vorgesehen ist. Korrespondierend zur Anzahl und geometrischen Verteilung der Dosierkavitäten sind in der Anlagefläche Dosieröffnungen ausgebildet, in die vom Pulvervorratsbehälter ausgehende Pulverkanäle münden. Die Dosierkavitäten weisen in der Ebene der Transferfläche jeweils eine offene Seite und gegenüberliegend dazu jeweils einen Boden auf, wobei der Boden der jeweiligen Dosierkavität zumindest abschnittsweise durch ein luftdurchlässiges Filterelement gebildet ist. Im Dosierorgan ist ein zentraler Druckkanal ausgebildet, der mit den Dosierkavitäten durch die Filterelemente hindurch druckübertragend verbunden ist. Die Zielkavitäten und die geometrisch darauf abgestimmte Dosiereinrichtung bilden zusammen das erfindungsgemäße Dosiersystem.

Im zugehörigen erfindungsgemäßen Verfahren sind folgende Verfahrensschritte vorgesehen: Zunächst wird das Dosierorgan mit seiner ebenen Transferfläche dichtend an die ebene Anlagefläche der Dosierstation angelegt. Durch den zentralen Druckkanal wird durch die Filterelemente, die Dosierkavitäten und die Pulverkanäle hindurch eine negative Druckdifferenz auf das Pulver im Pulvervorratsbehälter aufgebracht, in dessen Folge die Dosierkavitäten durch die ihnen zugeordneten Pulverkanäle aus dem Pulvervorratsbehälter gleichzeitig mit dem Pulver befüllt werden, wobei das Pulver an den Filterelementen zurückgehalten wird. Unter Aufrechterhaltung einer negativen Druckdifferenz wird das Dosierorgan anschließend von der Dosierstation zur Füllstation verfahren, wobei die Dosierkavitäten in Überdeckung mit zugeordneten Zielkavitäten gebracht werden. Die negative Druckdifferenz wird aufgehoben und das Pulver aus den Dosierkavitäten in die jeweils zugeordnete Zielkavitäten gebracht. Schließlich wird das Dosierorgan zurück zur Dosierstation verfahren.

Abweichend zu Membranfüllern nach dem Stand der Technik befinden sich die Filterelemente und die Pulverkanäle nicht auf der gleichen Seite der Zielkavität, sondern auf sich gegenüberliegenden Seiten davon. Es gibt keine mechanische Verbindung und auch keine geometrische Interaktion zwischen den Filterelementen und den Pulverkanälen, so dass diese unabhängig voneinander ausgestaltet werden können. Durch das Filterelement hindurch kann das Pulver vollflächig angesaugt werden, so dass auch bei geometrisch komplexen Grundrissen der Dosierkavitäten eine randvolle Befüllung möglich ist. Gleichzeitig wirkt die Dosierkavität aber nur als Zwischenvolumen, welches in seinem Betrag vom Volumen der Zielkavität abweichen kann. Damit ist ohne Weiteres eine Teilbefüllung der Zielkavitäten möglich. Die ebene Ausgestaltung von Transferfläche und Anlagefläche erlaubt eine passgenaue und dichte gegenseitige Anlage, so dass das Pulver exakt und ohne Fehlströmungen durch die zugeordneten Pulverkanäle angesaugt werden kann. Durch die ebene Ausgestaltung bestehen außerdem keine geometrischen Einschränkungen in der flächigen Verteilung der Dosierkavitäten, so dass diese in Anzahl und geometrischer Verteilung korrespondierend zur ebenen Anordnung der Zielkavitäten positioniert werden können. Die gemeinsame Druckbeaufschlagung durch den zentralen Druckkanal erlaubt ein gleichzeitiges Dosieren mit an jeder Dosierkavität zumindest näherungsweise gleicher Druckdifferenz. Das Gleiche gilt auch für die gleichzeitige Übergabe der dosierten Pulverteilmengen aus den Dosierkavitäten in die Zielkavitäten. Die gewünschte Dosier- und Füllmenge lässt sich leicht durch Volumenanpassung der Dosierkavitäten beispielsweise durch deren Ausbildung als Tausch- bzw. Formatteil realisieren, ohne dass Änderungen an den Zielkavitäten erforderlich sind.

In einer bevorzugten Weiterbildung ist das Dosierorgan um eine Rotationsachse schwenkbar, wobei das Dosierorgan in einer 0°-Rotationsposition mit seiner ebenen Transferfläche nach oben weisend unterhalb der Dosierstation liegt, und wobei das Dosierorgan in einer 180°-Rotationsposition mit seiner ebenen Transferfläche nach unten weisend oberhalb der Füllstation liegt. Insbesondere sind mehrere Dosierorgane um die Rotationsachse herum angeordnet und für ein sequenzielles Anfahren der Dosierstation und der Füllstation zu einer gemeinsamen rotierbaren Einheit zusammengefasst. In getakteten Rotationsschritten lassen sich kurze Zyklenzeiten erzielen. Bei der entsprechenden Anordnung von mehreren Dosierorganen sind verschiedene Vorgänge wie Dosieren, Füllen, Inspizieren, Reinigen oder dergleichen in einzelnen Taktschritten gleichzeitig durchführbar.

In einer bevorzugten Ausführungsform ist das Dosierorgan in Richtung einer Hubachse linear gegen die ebene Anlagefläche der Dosierstation verfahrbar und von ihr abhebbar. Im zugehörigen Betriebsverfahren wird das Dosierorgan zunächst in Richtung der Hubachse linear gegen die ebene Anlagefläche der Dosierstation verfahren, wobei dann durch Beaufschlagung mit einer negativen Druckdifferenz die Dosierkavitäten mit Pulver befüllt werden. Die negative Druckdifferenz führt nicht nur dazu, dass die Dosierkavitäten mit dem Pulver befüllt werden. Sie bewirkt auch, dass das Dosierorgan gegen die Anlagefläche der Dosierstation gesaugt wird. Dies steigert einerseits die Dichtwirkung an den gegeneinander gepressten Flächen. Andererseits wird aber ein Abheben des Dosierorgans von der Anlagefläche der Dosierstation erschwert. Um im Anschluss an den Dosiervorgang den Abhebevorgang zu erleichtern, wird kurzzeitig die negative Druckdifferenz aufgehoben. Nach dem Abhebevorgang wird unter erneuter Aufbringung und Aufrechterhaltung einer negativen Druckdifferenz das Dosierorgan von der Dosierstation zur Füllstation verfahren. Die negative Druckdifferenz verhindert, dass das Pulver während des Verfahr- bzw. Rotationsvorganges aus den Dosierkavitäten fällt.

Je nach Eigenschaften des verwendeten Pulvers kann an der Dosierstation eine gleichmäßige negative Druckdifferenz für den Dosiervorgang ausreichen. Bevorzugt wird an der Dosierstation für die Befüllung der Dosierkavitäten mit dem Pulver eine impulsartige negative Druckdifferenz aufgebracht. Dies steigert kurzzeitig die Fließfähigkeit des Pulvers und damit eine exakte Füllung der Dosierkavitäten.

Für die Überführung des Pulvers aus den Dosierkavitäten in die Zielkavitäten am Ort der Füllstation kommen verschiedene Möglichkeiten in Betracht. Geeignet sind ein Druckstoß mit positiver Druckdifferenz, ein zwischen positiver und negativer Druckdifferenz oszillierender Luftdruck, ein mechanischer Impuls oder eine mechanische Schwingung, welche auf das in den Dosierkavitäten befindliche Pulver aufgebracht wird. Hierbei kann es je nach Bedarf zweckmäßig sein, dass das Dosierorgan an der Füllstation mit geringem Abstand zu den Zielkavitäten liegt. Hierdurch wird beispielsweise eine Verdrängung der in den Zielkavitäten befindlichen Luft durch das eintretende Pulver begünstigt. Insbesondere wird aber an der Füllstation das Dosierorgan mit seiner ebenen Transferfläche dichtend an Siegelflächen der Zielkavitäten angedrückt. Hierdurch ist sichergestellt, dass das dosierte Pulver ausschließlich in die Zielkavität gelangt, ohne dass Teilmengen davon ungewollt austreten bzw. auf die Siegelflächen gelangen. Die Dosiergenauigkeit ist gesteigert, während außerdem ein späteres dichtes Absiegeln der Zielkavitäten mit einer Siegelfolie zuverlässig möglich ist.

In einer bevorzugten Ausführungsform ist die ebene Transferfläche des Dosierorgans zumindest abschnittsweise durch ein elastisch nachgiebiges, geschlossen um die Dosierkavitäten umlaufendes Dichtmaterial gebildet. Das Dichtmaterial kann luft- und pulverdicht oder luftdurchlässig porös, aber dicht gegen Pulverdurchtritt sein. Außerdem ist eine Kombination beider Eigenschaften beispielsweise durch Ausgestaltung als Schaumstoff möglich, wobei je nach Kompressionszustand der Poren eine Luft- und Pulverdichtigkeit oder aber eine Luftdurchlässigkeit bei gleichzeitiger Pulverdichtigkeit erzielt wird. Bevorzugt weisen dabei die Dosierkavitäten einen unnachgiebigen, umlaufenden Rand auf, wobei der Rand zur direkten Anlage an der Anlagefläche der Dosierstation vorgesehen ist, und wobei das Dichtmaterial um den Rand herumläuft. Das elastische Dichtmaterial stellt insbesondere unter Einwirkung einer mechanischen Anpresskraft sicher, ggf. unterstützt durch Saugkraft infolge der aufgebrachten negativen Druckdifferenz, dass keine Fehlluft im Bereich der umlaufenden Ränder angesaugt wird, sondern dass ausschließlich eine Pulveransaugung durch den jeweiligen Pulverkanal erfolgt. Gleichzeitig wird ein Pulveraustritt vermieden. Der im Vergleich dazu starre, unnachgiebige Rand bewirkt eine exakte räumliche Begrenzung der Dosierkavität mit einem ebenso exakt bestimmten Volumen, was die volumetrische Dosiergenauigkeit erhöht. Im Falle der Luftdurchlässigkeit bei gleichzeitiger Pulverdichtigkeit kann insbesondere an der Füllstation erreicht werden, dass die vom eingefüllten Pulver aus den Zielkavitäten verdrängte Luft entweichen kann, während gleichzeitig ein Pulveraustritt aus den Zielkavitäten bzw. eine unerwünschte Beaufschlagung der Siegelflächen mit Pulver verhindert wird.

Ausführungsbeispiele der Erfindung sind nachfolgend anhand der Zeichnung näher beschrieben. Es zeigen:
- Fig. 1: in einer schematischen Übersichtsdarstellung ein Ausführungsbeispiel eines erfindungsgemäßen Dosiersystems mit einer Dosierstation, mit einer Füllstation, in der eine Anzahl von in einer gemeinsamen Ebene angeordneten Zielkavitäten gehalten ist, und mit mindestens einem, hier vier Dosierorganen, welche zyklisch von der Dosierstation zur Füllstation und wieder zurück verfahrbar sind;
- Fig. 2: in einer geschnittenen Frontansicht die Anordnung nach Fig. 1 mit Einzelheiten zur Ausgestaltung der Dosierstation und der zu einer gemeinsam beweglichen Einheit zusammengefassten Dosierorgane;
- Fig. 3: in einer vergrößerten Detailansicht die Anordnung nach Fig. 2 im Bereich der Dosierstation mit Einzelheiten zu deren Zusammenwirken mit dem darunter befindlichen Dosierorgan;
- Fig. 4: in einer vergrößerten Detailansicht die Anordnung nach Fig. 2 im Bereich der Füllstation mit Einzelheiten zu deren Zusammenwirken mit dem darüber befindlichen Dosierorgan;
- Fig. 5: eine Variante der Anordnung nach Fig. 3 mit einem gerundet ausgestalteten Pulverkanal;
- Fig. 6: eine weitere Variante der Anordnung nach den Fig. 3 und 5 mit einem gestuft ausgestalteten Pulverkanal.

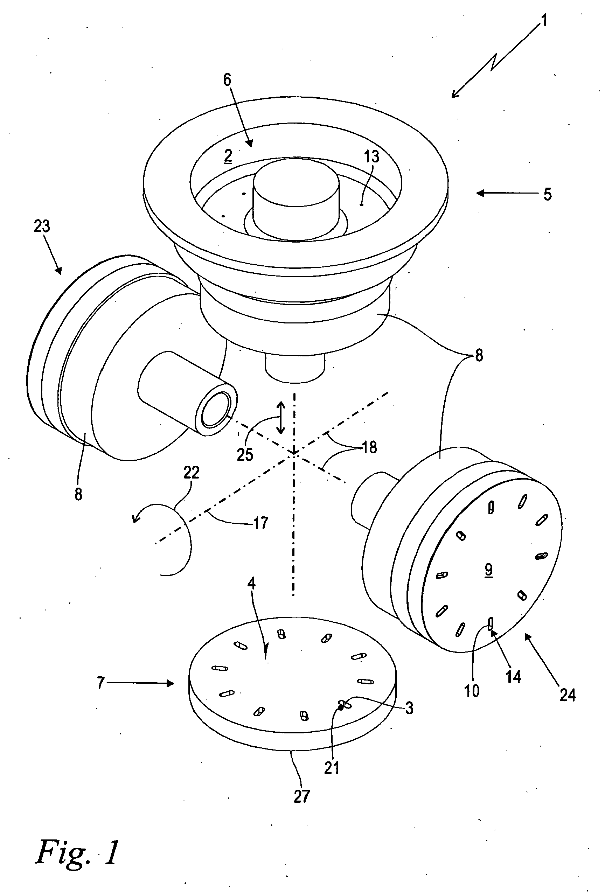

Fig. 1 zeigt in einer schematischen Übersichtsdarstellung ein erstes Ausführungsbeispiel eines erfindungsgemäßen Dosiersystems, welches eine Dosiereinrichtung 1 sowie eine zugeordnete Anzahl von in einer gemeinsamen Ebene 4 angeordneten Zielkavitäten 3 umfasst. Die mehreren Zielkavitäten 3 liegen demnach nicht auf einer gemeinsamen Linie, sondern spannen die Ebene 4 in zwei verschiedenen horizontalen Raumrichtungen auf, so dass also die Ebene 4 horizontal bzw. quer zur Gewichtskraftrichtung ausgerichtet ist. Ferner läuft um die anfänglich einseitig offenen Zielkavitäten 3 eine Siegelfläche 21 um. Auf die Siegelfläche 21 wird nach Befüllung mit einem Pulver 2 eine nicht dargestellte Deckfolie aufgesiegelt, wodurch die befüllten Zielkavitäten 3 dicht verschlossen werden.

Die hier zehn Zielkavitäten 3 sind hier beispielhaft ringförmig in einer ebenen DPI-Disc 27 (Dry Powder Inhaler Disc) ausgebildet. Eine solche DPI-Disc 27 wird in Inhalationsgeräten zur pulmonalen Verabreichung von pharmazeutischem Pulver 2 eingesetzt. DPI-Discs weisen typischerweise zehn bis einundsechzig Zielkavitäten 3 auf. Anstelle der gezeigten ringförmigen Anordnung der Zielkavitäten 3 ist auch jede beliebige andere geometrische Anordnung beispielsweise in Rechteck-, Polygon- oder Matrixform denkbar, solange die Zielkavitäten 3 in einer gemeinsamen Ebene 4 liegen. Außerdem ist es nicht zwingend erforderlich, dass die Zielkavitäten 3 in einem gemeinsamen Bauteil, hier in der DPI-Disc 27 ausgeformt sind. Es sind auch Zielkavitäten 3 als separate Behälter oder in Bauteilen zusammengefasste Gruppen davon denkbar. Im Rahmen der Erfindung lässt sich auch eine beliebige andere Anzahl von Zielkavitäten 3 gleichzeitig dosiert befüllen. Die Zielkavitäten 3 weisen hier beispielhaft einen länglichen, ovalen Grundriss auf. Jeder andere Grundriss der Zielkavität 3 ist ebenso im Rahmen der Erfindung einsetzbar. Außerdem ist die Erfindung nicht auf die Dosierung von pharmazeutischem Pulver zur pulmonalen Verabreichung beschränkt. Ebenso ist die Erfindung auf eine Dosierung von anderen pharmazeutischen Pulvern oder pulvrigen Nahrungsergänzungsmitteln anwendbar, wobei der hier gewählte Begriff des Pulvers 2 auch Granulate oder dergleichen umfasst.

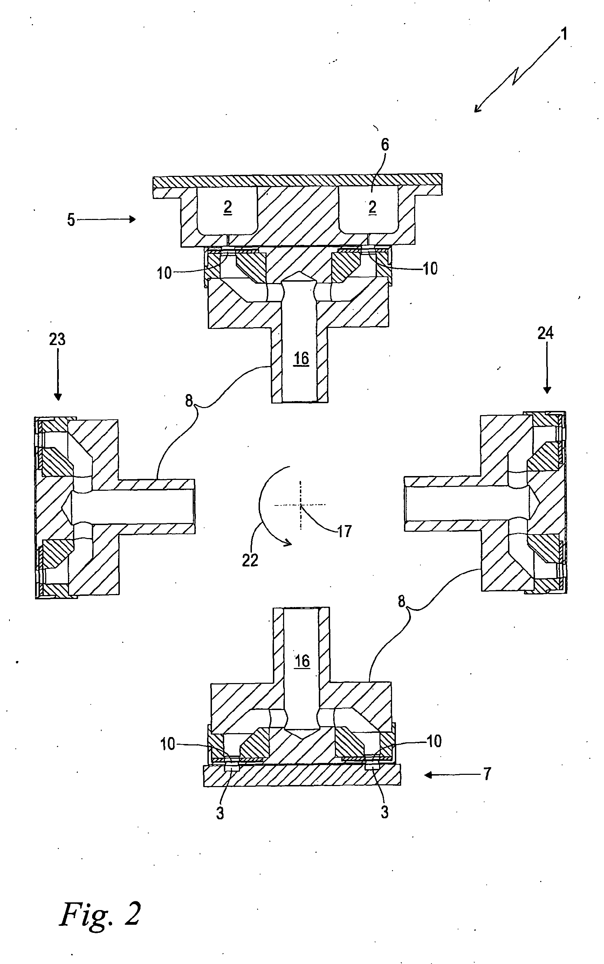

Die Dosiereinrichtung 1 umfasst eine Dosierstation 5 mit einem Pulvervorratsbehälter 6, in dem eine Vorratsmenge von Pulver 2 bereitgehalten wird. Des Weiteren umfasst die Dosiereinrichtung 1 noch eine Füllstation 7 sowie mindestens ein bewegliches Dosierorgan 8. Im gezeigten Ausführungsbeispiel sind entsprechend der Darstellung nach Fig. 2 insgesamt vier Dosierorgane 8 vorgesehen, von denen das untere Dosierorgan 8 zur besseren Darstellung der DPI-Disc 27 in Fig. 1 nicht gezeigt ist. Die Füllstation 7 ist bezogen auf die Gewichtskraftrichtung unterhalb der Dosierstation 5 angeordnet. Optional können noch eine oder mehrere Zwischenstationen 23, 24 vorgesehen sein, deren Funktion weiter unten näher beschrieben wird. Das mindestens eine Dosierorgan 8 ist zyklisch von der Dosierstation 5 zur Füllstation 7 und wieder zurück verfahrbar, was durch einen Linearhub oder dergleichen realisiert werden kann. Im gezeigten bevorzugten Ausführungsbeispiel ist das Dosierorgan 8 um eine Rotationsachse 17 schwenkbar, wobei das Dosierorgan 8 in einer oberen 0°-Rotationsposition unterhalb der Dosierstation 5 liegt, und wobei das Dosierorgan 8 in einer unteren 180°-Rotationsposition oberhalb der Füllstation 7 liegt. In entsprechenden Winkel-Zwischenschritten können optional weitere Zwischenstationen 23, 24 angefahren werden. Im gezeigten Ausführungsbeispiel sind mehrere, hier beispielhaft vier Dosierorgane 8 um die Rotationsachse 17 herum angeordnet und für ein sequenzielles Anfahren der Dosierstation 5, der Füllstation 7 sowie der optionalen Zwischenstationen 23, 24 zu einer gemeinsam um die Rotationsachse 17 rotierbaren Einheit zusammengefasst. Außerdem ist jedes Dosierorgan 8 in Richtung einer Hubachse 18 entsprechend einem Doppelpfeil 25 linear verfahrbar, wobei jede Hubachse 18 jedes Dosierorgans 8 radial zur Rotationsachse 17 liegt.

Der Darstellung nach Fig. 1 ist noch entnehmbar, dass die Dosierorgane 8 jeweils eine ebene Transferfläche 9 aufweisen, in der korrespondierend zur Anzahl und zur geometrischen Verteilung der Zielkavitäten 3 mehrere Dosierkavitäten 10 eingearbeitet sind. In der Ebene der Transferfläche 9 weisen die Dosierkavitäten 10 jeweils eine vom Grundkörper des Dosierorgans 8 fort weisende offene Seite 14 auf. Bevorzugt, aber nicht zwingend weisen die Dosierkavitäten 10 im Bereich ihrer offenen Seite 14 den gleichen Grundriss wie die Zielkavitäten 3 auf. "Korrespondierend zur Anzahl und zur geometrischen Verteilung bzw. Anordnung" bedeutet hier, dass je eine Dosierkavität 10 an der Füllstation 7 mit je einer Zielkavität 3 in Überdeckung gebracht werden kann. Hierzu sind die Dosierkavitäten 10 ebenso wie die Zielkavitäten 3 ringförmig mit gleicher Winkelteilung und mit gleichem Radius in der Transferfläche 34 ausgebildet. Analoges gilt bei einer abweichenden Verteilung der Zielkavitäten 3 auf der Ebene 4. Obiges bedeutet aber nicht, dass notwendig die gleiche Anzahl von Dosierkavitäten 10 und Zielkavitäten 3 vorhanden sein muss. Es kann auch zweckmäßig sein, dass ausgehend von der Anzahl der Zielkavitäten 3 ein n-facher Teiler davon (1/2, 1/3 oder dgl.) als Anzahl von Dosierkavitäten 10 vorgesehen ist. In diesem Fall können in einem ersten Takt ein erster Teil der Zielkavitäten 3 und in einem oder mehreren nachfolgenden Takten die übrigen Zielkavitäten 3 befüllt werden.

Weiter ist der Darstellung von Fig. 1 noch entnehmbar, dass im Boden des Pulvervorratsbehälters 6 eine Anzahl von Pulverkanälen 13 ausgebildet ist, deren Funktion weiter unten näher beschreiben wird. Jeder Dosierkavität 10 ist mindestens je ein Pulverkanal, hier genau ein Pulverkanal 13 zugeordnet. Es kann aber auch zweckmäßig sein, dass jeder Dosierkavität 10 zwei oder mehr Pulverkanäle 13 zugeordnet sind.

Fig. 2 zeigt in einer geschnittenen Frontansicht die Anordnung nach Fig. 1 mit Einzelheiten zur Ausgestaltung der Dosierstation 5 und der zu einer gemeinsam beweglichen, hier gemeinsam um die Rotationsachse 17 schwenkbaren Einheit zusammengefassten Dosierorgane 8. Entsprechend der ringförmigen Anordnung der Dosierkavitäten 10 ist auch der Pulvervorratsbehälter 6 ringförmig ausgebildet, wobei bei der Platzierung des Dosierorgans 8 unterhalb der Dosierstation 5 die ringförmig angeordneten Dosierkavitäten 10 unterhalb des ebenfalls ringförmig ausgebildeten Pulvervorratsbehälters 6 zu liegen kommen. Bei einer abweichenden Anordnung der Dosierkavitäten 10 ist eine analoge Ausgestaltung des Pulvervorratsbehälters 6 zweckmäßig.

Sobald das jeweilige Dosierorgan 8 infolge der Schwenkbewegung um die Rotationsachse 17 entsprechend dem Pfeil 22 die untere Füllstation 7 erreicht hat, liegen die Dosierkavitäten 10 exakt über je einer zugeordneten Zielkavität 3.

Weitere Einzelheiten zur Zusammenschau der Fig. 1 und 2 sind weiter unten noch beschrieben.

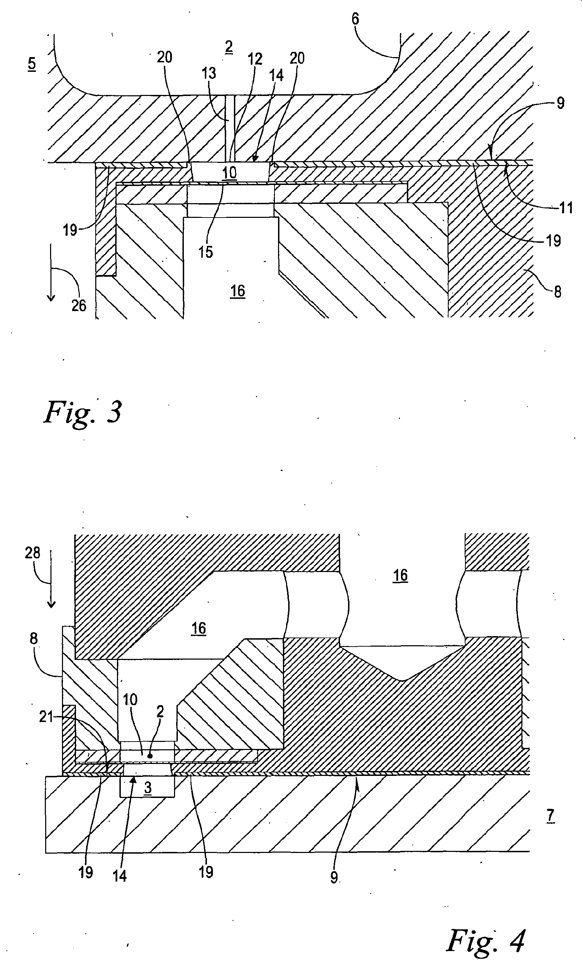

Fig. 3 zeigt in einer vergrößerten Detailansicht die Anordnung nach Fig. 2 im Bereich der Dosierstation 5. Es ist erkennbar, dass die Dosierstation 5 auf ihrer bezogen auf die Gewichtskraftrichtung unteren Seite eine ebene Anlagefläche 11 aufweist, die horizontal ausgerichtet ist, und die zur dichtenden Anlage des Dosierorgans 8 mittels seiner Transferfläche 9 vorgesehen ist. Unter gleichzeitigem Bezug auf die Fig. 1 und 2 wird deutlich, dass die Transferfläche 9 des jeweiligen Dosierorgans 8 senkrecht zu der von der Rotationsachse 17 ausgehenden Radialrichtung liegt, so dass die Transferfläche 9 in der hier gezeigten oberen 0°-Rotationsposition ebenfalls horizontal liegt. Während des Rotationsvorganges der Dosierorgane 8 besteht ein radialer Abstand zwischen der Transferfläche 9 und der Anlagefläche 11. Sobald das Dosierorgan 8 die obere 0°-Rotationsposition entsprechend Fig. 3 erreicht hat, wird es entsprechend dem Doppelpfeil 25 in Richtung der zugehörigen Hubachse 18 (Fig. 1) von unten nach oben gegen die Dosierstation 5 verfahren, in dessen Folge die Transferfläche 9 des Dosierorgans 8 dichtend an die Anlagefläche 11 der Dosierstation 5 angedrückt wird. Für eine zumindest näherungsweise luftdichte Anlage ist die ebene Transferfläche 9 des Dosierorgans 8 zumindest abschnittsweise durch ein elastisch nachgiebiges, geschlossen um die jeweiligen Dosierkavitäten 10 umlaufendes Dichtmaterial 19, hier beispielhaft in Form einer flachen Scheibe aus elastischem Silikon gebildet.

In einer bevorzugten Ausführungsform ist das Dichtmaterial 19 massiv und damit insgesamt luftdicht und kann dicht bezüglich Luft- und Pulverdurchtritt gegen die ebene Anlagefläche 11 der Dosierstation 5 bzw. gegen die Siegelfläche 21 der Zielkavitäten 3 in der Füllstation 7 gepresst werden. Eine weitere bevorzugte Möglichkeit besteht im Einsatz eines luftdurchlässigen, aber für das Pulver 2 undurchlässigen porösen Dichtmaterials 19 im weiter unten näher beschriebenen Zusammenspiel mit der Füllstation 7. Beide Eigenschaften können auch miteinander kombiniert werden, indem beispielsweise ein aufgeschäumtes Dichtmaterial 19 zum Einsatz kommt. Bei entsprechend hohem Anpressdruck werden die Poren des elastischen Dichtmaterials 19 zusammengedrückt, wodurch es undurchlässig für Luft und Pulver 2 wird. Bei geringerem Anpressdruck bleiben die Poren bestehen, wodurch das gleiche Dichtmaterial 19 zwar luftdurchlässig, aber für das Pulver 2 undurchlässig ist.

Es kann zweckmäßig sein, die Transferfläche 9 vollständig aus dem Dichtmaterial 19 auszubilden. Im gezeigten bevorzugten Ausführungsbeispiel weisen die Dosierkavitäten 10 auf ihrer der Rotationsachse 17 abgewandten, hier der Dosierstation 5 zugewandten Seite jeweils eine offene Seite 14 auf, die von einem kragenförmigen, um die offene Seite 14 der jeweiligen Dosieröffnung 12 umlaufenden Rand 20 umschlossen ist. Das Dichtmaterial 19 läuft bezogen auf die Umfangsrichtung der offenen Seite 14 um den kragenförmigen Rand 20 um, so dass es nicht zwischen dem Rand 20 und der Anlagefläche 11 zu liegen kommt. Infolge des vorgeschriebenen Anpressdruckes wird das elastisch nachgiebige Dichtmaterial 19 zusammengedrückt, bis der im Vergleich dazu starre, hier metallische Rand 20 direkt an der Anlagefläche 11 der Dosierstation 5 anliegt. Hierbei bildet der umlaufende Rand 20 mit hoher lokaler Flächenpressung eine umlaufend und dicht anliegende Begrenzungskante für die jeweilige Dosierkavität 10.

Fig. 3 zeigt des Weiteren, dass im Boden des Pulvervorratsbehälters 6 korrespondierend zu jeder Dosierkavität 10 jeweils mindestens ein Pulverkanal 13 ausgebildet ist, der vom Pulvervorrat des Pulvervorratsbehälters 6 zur unteren Anlagefläche 11 führt und dort in einer nach unten offenen Dosieröffnung 12 mündet. Auch hier sind die Dosieröffnungen 12 korrespondierend zur Anzahl und geometrischen Verteilung der Dosierkavitäten 10 in der Anlagefläche 11 ausgebildet bzw. verteilt. Diese Definition beinhaltet aber auch die Möglichkeit, dass jeder Dosierkavität 10 zwei oder mehr Dosieröffnungen 12 bzw. Pulverkanäle 13 zugeordnet sein können.

Weiter oben wurde schon erwähnt, dass die Dosieröffnungen 12 außen bzw. in der 0°-Rotationsposition nach Fig. 3 oben jeweils eine offene Seite 14 aufweisen. Gegenüberliegend dazu, also hier unten bzw. bezogen auf die senkrecht zur Rotationsachse 17 (Fig. 1) liegende Radialrichtung innen, sind die Dosierkavitäten 10 durch einen Boden begrenzt. Der Boden der Zielkavitäten 10 ist zumindest abschnittsweise, hier vollflächig durch ein luftdurchlässiges Filterelement 15 ausgebildet. Das Filterelement 15 ist bezüglich seiner Durchlässigkeit auf das Pulver 2 derart abgestimmt, dass zwar Luft, nicht aber Pulverpartikel hindurchtreten können.

Unter erneutem Bezug auf Fig. 2 ist noch erkennbar, dass in jedem Dosierorgan 8 je ein zentraler Druckkanal 16 ausgebildet ist, der über entsprechende Verzweigungen mit allen Dosierkavitäten 10 durch die Filterelemente 15 hindurch luftdruckübertragend verbunden ist. Eine Besonderheit des Druckkanals 16 besteht darin, dass er auf der Mittelachse des jeweiligen Dosierorgans 8 angeordnet ist, während die Dosierkavitäten 10 ringförmig um die genannte Mittelachse platziert sind. Hierdurch weisen alle zu den jeweiligen Dosierkavitäten 10 führenden Verzweigungen des Druckkanals 16 zumindest näherungsweise die gleiche Länge auf, in dessen Folge mittels des zentralen Druckkanals 16 an allen Dosierkavitäten 10 zumindest näherungsweise die gleiche Druckdifferenz aufgebracht werden kann.

Ausgehend von Fig. 3 unter gleichzeitigem Bezug auf die Darstellung nach den Fig. 1 und 2 wird folgendes erfindungsgemäße Verfahren ausgeführt:
Zunächst wird ein Dosierorgan 8 zur Dosierstation 5 verfahren und dort infolge der oben genannten Hubbewegung in Richtung der Hubachse 18 mit seiner ebenen Transferfläche 9 dichtend an die ebene Anlagefläche 11 der Dosierstation 5 angelegt. Hierbei wird die offene Seite 14 der Dosierkavität 10 durch die Anlagefläche 11 abgedeckt, so dass in Verbindung mit umlaufenden Umfangswänden und dem Filterelement 15 ein geschlossenes Volumen der jeweiligen Dosierkavität 10 gebildet bzw. definiert ist. Durch die genannte Hubbewegung wird das Dichtmaterial zwischen dem Grundkörper des Dosierorgans 8 und der Anlagefläche 11 der Dosierstation 5 zusammengedrückt, wodurch eine gegen Luft- und Pulverdurchtritt dichte Verbindung zwischen dem Dosierorgan 8 und der Dosierstation 5 geschaffen ist. Im Falle des oben erwähnten luftdurchlässig porösen Dichtmaterials 19 kann dieses so weit zusammengedrückt werden, dass Luft- und Pulverdichtigkeit eintritt. Alternativ kann aber auch eine geringere Kompression des luftdurchlässig porösen Dichtmaterials 19 ausreichen, wobei dann die Luft- und Pulverdichtigkeit allein durch den Anpressdruck des Randes 20 an der Anlagefläche 11 hergestellt wird.

In dieser Position wird nun durch den zentralen Druckkanal 16, durch die Filterelemente 15, die Dosierkavitäten 10 und die Pulverkanäle 13 hindurch eine negative Druckdifferenz auf das Pulver 2 im Pulvervorratsbehälter 6 aufgebracht. Der hier gewählte Begriff der "negativen Druckdifferenz" bedeutet zunächst allgemein, dass im Druckkanal 16 ein geringerer Luftdruck herrscht als außenseitig des Pulvervorrats. Umgekehrt bedeutet eine "positive Druckdifferenz", dass im Druckkanal 16 ein höherer Druck als im Pulvervorratsbehälter 6 herrscht. Eine negative Druckdifferenz kann beispielsweise dadurch herbeigeführt werden, dass der Pulvervorratsbehälter 6 geschlossen und innen mit einem Überdruck beaufschlagt wird, während im Druckkanal 16 relativ dazu ein geringerer Druck, beispielsweise atmosphärischer Umgebungsdruck herrscht. Bevorzugt herrscht im Pulvervorratsbehälter 6 atmosphärischer Umgebungsdruck, während mittels einer nicht dargestellten Unterdruckquelle (Vakuumpumpe) ein Unterdruck im Druckkanal 16 ausgebildet wird. In jedem Falle führt die negative Druckdifferenz dazu, dass das im Pulvervorratsbehälter 6 bereitgestellte Pulver durch die Pulverkanäle 13 in die Dosierkavitäten 10 gesaugt und dort an den Filterelementen 15 zurückgehalten wird, während mitgeführte Luft und die zuvor in den Dosierkavitäten befindliche Luft durch die Filterelemente 15 und den Druckkanal 16 entweicht bzw. abgesaugt wird. Hierdurch werden alle Dosierkavitäten 10 eines einzelnen Dosierorgans 8 gleichzeitig vollständig mit Pulver 2 befüllt und dabei infolge des definierten Volumens der einzelnen Dosierkavitäten 10 volumetrisch exakt dosiert.

Es kann zweckmäßig sein, während der Befüllung der Dosierkavitäten 10 eine kontinuierliche, gleichbleibende negative Druckdifferenz aufrechtzuerhalten. Bevorzugt wird mindestens eine impulsartige negative Druckdifferenz aufgebracht. Ebenso kann es zweckmäßig sein, einen oder mehrere negative Druckimpulse mit einer kontinuierlichen negativen Grunddruckdifferenz zu kombinieren.

Im Anschluss an die Befüllung der Dosierkavitäten 10 wird das an der Dosierstation 5 anliegende Dosierorgan 8 in Richtung der Hubachse 18 (Fig. 1) entsprechend einem Pfeil 26 (Fig. 3) nach unten verfahren, so dass die Transferfläche 9 von der Anlagefläche 11 der Dosierstation 5 abgehoben wird. Alternativ kann es auch zweckmäßig sein, das Dosierorgan zunächst in seiner Position zu belassen und stattdessen die Dosierstation anzuheben. In beiden Fällen kommt es darauf an, dass ein abhebender Relativhub zwischen Dosierorgan 8 und Dosierstation 5 stattfindet. Für den genannten Abhebevorgang kann es zweckmäßig sein, die negative Druckdifferenz zeitweilig aufzuheben, damit der Abhebevorgang nicht durch die aus der Druckdifferenz resultierenden Anpresskräfte behindert wird. Sobald aber das Dosierorgan 8 mit seiner Transferfläche 9 von der Anlagefläche 11 der Dosierkavität 10 abgehoben ist, wird erneut eine negative Druckdifferenz aufgebracht. Unter Aufrechterhaltung dieser negativen Druckdifferenz wird das Dosierorgan 8 von der Dosierstation 5 zur Füllstation 7 verfahren, wie es in Fig. 2 erkennbar und in der vergrößerten Detailansicht nach Fig. 4 in Einzelheiten dargestellt ist. Die Aufrechterhaltung der negativen Druckdifferenz verhindert während der dazwischen liegenden Rotationsbewegung, dass das in den Dosierkavitäten 10 befindliche Pulver 2 aus den offenen Seiten 14 herausfällt.

Beim Erreichen der Füllstation 7 bzw. der 180°-Rotationsposition entsprechend der Darstellung nach Fig. 4 weisen die Transferfläche 9 des Dosierorgans 8 und die in gleicher Ebene liegenden offenen Seiten 14 der Dosierkavitäten 10 in Gewichtskraftrichtung nach unten, wobei die Dosierkavitäten 10 genau über je einer Zielkavität 13 zu liegen kommen. Hiervon ausgehend erfolgt nun eine Befüllung der Zielkavitäten 3, wozu das dosierte Pulver 2 aus den Dosierkavitäten 10 in die jeweils zugeordneten Zielkavitäten 3 überführt wird. Hierbei kann es zweckmäßig sein, abweichend vom Dosiervorgang nach Fig. 3 auf eine Hubbewegung in Richtung der Hubachse 18 (Fig. 1) zu verzichten, so dass ein Abstand zwischen der Transferfläche 9 und dem oberen Rand der Zielkavitäten 3 verbleibt. In einer bevorzugten Ausführungsform wird das Dosierorgan 8 in Richtung der Hubachse 18 (Fig. 1) entsprechend einem Pfeil 28 (Fig. 4) auf die Zielkavitäten 3 abgesenkt, wobei die ebene Transferfläche 9 des Dosierorgans 8 dichtend an Rändern, insbesondere an Siegelflächen 21 der Zielkavitäten angedrückt wird. Auch hier kommt es vor allem auf einen Relativhub an, was auch durch ein Anheben der Füllstation 7 herbeigeführt werden kann. Die Transferfläche 9 und insbesondere deren elastisches Dichtmaterial 19 decken dabei die genannten Ränder bzw. die Siegelflächen 21 der Zielkavitäten 3 ab, so dass hierauf kein Pulver gelangen kann. Auch hier kann ein elastisches Dichtmaterial 19 in luft- und pulverdichter Form zum Einsatz kommen. Im bevorzugten Fall des oben schon beschriebenen luftdurchlässig porösen Dichtmaterials 19, sofern dieses nicht in einen luftdichten Zustand komprimiert wird, kann aber erreicht werden, dass die vom eingefüllten Pulver 2 aus den Zielkavitäten 3 verdrängte Luft durch das Dichtmaterial 19 hindurch entweichen kann, während das Dichtmaterial 19, welches gleichzeitig für das Pulver 2 undurchlässig ist, einen Pulveraustritt aus den Zielkavitäten 3 bzw. eine unerwünschte Beaufschlagung der Siegelflächen 21 mit Pulver 2 verhindert.

Zur Überführung des Pulvers 2 aus den Dosierkavitäten 10 in die Zielkavitäten 3 kann nun über den Druckkanal 16 ein entsprechender Druckverlauf aufgebracht werden. Dies kann ein kurzer Druckstoß mit positiver Druckdifferenz oder ein zwischen positiver und negativer Druckdifferenz oszillierender Luftdruck sein. Alternativ oder zusätzlich kann es zweckmäßig sein, einen mechanischen Impuls oder eine mechanische Schwingung auf das in den Dosierkavitäten 10 befindliche Pulver 2 aufzubringen. In allen Fällen wird eine Lösung des Pulvers 2 in den Dosierkavitäten 10 bewirkt, so dass es von dort in die Zielkavitäten 3 unter Einwirkung der Gewichtskraft fällt. Nach einer optionalen Anhebebewegung entgegen dem Pfeil 28 (Fig. 4) wird nun das Dosierorgan 8 mittels einer Schwenk- bzw. Rotationsbewegung um die Rotationsachse 17 entsprechend dem Pfeil 22 (Fig. 1) zurück zur Dosierstation 5 verfahren, wo der oben beschriebene Dosier- und Befüllzyklus erneut beginnt.

Weiter oben wurden schon die optionalen Zwischenstationen 23, 24 entsprechend der Darstellung nach den Fig. 1 und 2 erwähnt. Die erste, in der Rotationsrichtung entsprechend dem Pfeil 22 zwischen der Dosierstation 5 und der Füllstation 7 liegende Zwischenstation 23 kann als Prüfstation ausgeführt sein, in der geprüft wird, ob tatsächlich alle Dosierkavitäten 10 ordnungsgemäß mit Pulver 2 befüllt sind. Die zweite, bezogen auf die Rotationsrichtung entsprechend dem Pfeil 22 zwischen der unteren Füllstation 7 und der oberen Dosierstation 5 liegende Zwischenstation 24 kann beispielsweise als Inspektionsstation ausgeführt sein, in der geprüft wird, ob sämtliche Dosierkavitäten 10 zuvor ihre Pulvermenge in der Füllstation 7 an die Zielkavitäten 3 übergeben haben, oder ob sich noch Restmengen von Pulver 2 in den Dosierkavitäten 10 befinden. Natürlich sind auch noch weitere Stationen beispielsweise in Form von Reinigungsstationen oder dergleichen im Rahmen der Erfindung einsetzbar.

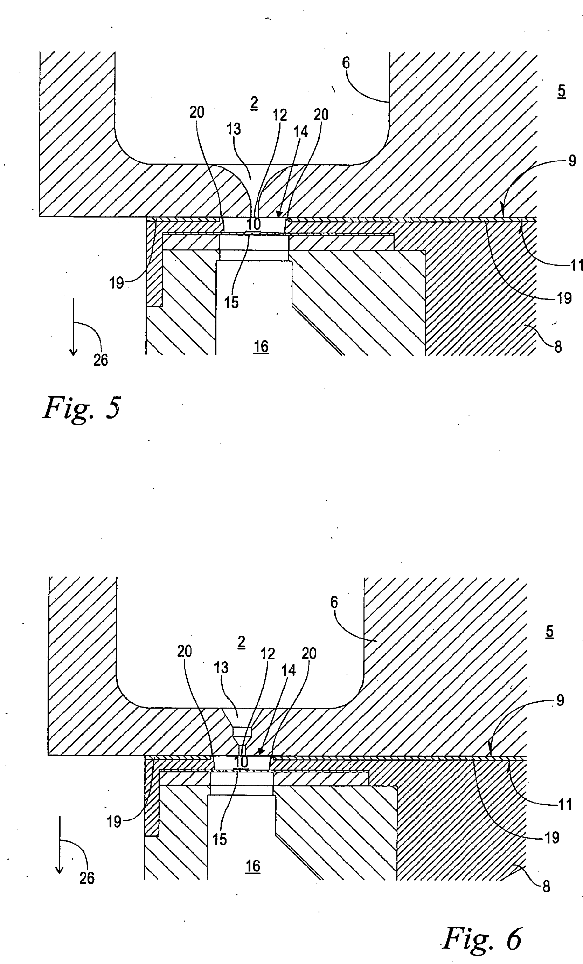

Die Fig. 5 und 6 zeigen noch Varianten der Anordnung nach Fig. 3, wobei in Fig. 3 der Pulverkanal 13 zylindrisch mit entlang der Lauflänge konstantem Querschnitt ausgebildet ist. Abweichend davon weist der Pulverkanal 13 im Ausführungsbeispiel nach Fig. 5 einen erweiterten, im Längsschnitt gerundeten Einlaufbereich auf, von dem ausgehend sich der Querschnitt des Pulverkanals 13 bis zur Dosieröffnung 12 hin verjüngt. Alternativ ist in Fig. 6 eine gestufte Querschnittsverjüngung des Pulverkanals 13 zur Dosieröffnung 12 hin mit konischen und zylindrischen Abschnitten vorgesehen. Hierdurch oder durch vergleichbare Längs- und Querschnittsformen lässt sich die Fließfähigkeit des Pulvers 2 durch den Pulverkanal 13 bei anliegender negativer Druckdifferenz verbessern, während gleichzeitig bei fehlender negativer Druckdifferenz ein unbeabsichtigtes Herausrieseln des Pulvers zuverlässig vermieden werden kann. In den übrigen Merkmalen und Bezugszeichen stimmen die Ausführungsbeispiele nach den Fig. 5 und 6 mit demjenigen nach den Fig. 1 bis 4 überein.

## Patentansprüche

1. Dosiereinrichtung (1) zum volumetrischen Dosieren eines insbesondere pharmazeutisches Pulvers (2) und zum gleichzeitigen Befüllen mehrerer Zielkavitäten (3) mit dem dosierten Pulver (2), wobei die mehreren Zielkavitäten (3) in einer gemeinsamen Ebene (4) angeordnet sind, umfassend eine Dosierstation (5) mit einem Pulvervorratsbehälter (6),
**dadurch gekennzeichnet, dass** die Dosiereinrichtung (1) des Weiteren eine Füllstation (7) und ein bewegliches Dosierorgan (8) umfasst, wobei das Dosierorgan (8) zyklisch von der Dosierstation (5) zur Füllstation (7) und wieder zurück verfahrbar ist, wobei das Dosierorgan (8) eine ebene Transferfläche (9) aufweist, in der korrespondierend zur Anzahl und geometrischen Verteilung der Zielkavitäten (3) mehrere Dosierkavitäten (10) eingearbeitet sind, wobei die Dosierstation (5) eine ebene Anlagefläche (11) aufweist, die zur dichtenden Anlage des Dosierorgans (8) mittels seiner Transferfläche (9) vorgesehen ist, wobei korrespondierend zur Anzahl und geometrischen Verteilung der Dosierkavitäten (10) Dosieröffnungen (12) in der Anlagefläche (11) ausgebildet sind, in die vom Pulvervorratsbehälter (6) ausgehende Pulverkanäle (13) münden, wobei die Dosierkavitäten (10) in der Ebene der Transferfläche (9) jeweils eine offene Seite (14) und gegenüberliegend dazu einen Boden aufweisen, wobei der Boden der jeweiligen Dosierkavität (10) zumindest abschnittsweise durch ein luftdurchlässiges Filterelement (15) gebildet ist, wobei im Dosierorgan (8) ein zentraler Druckkanal (16) ausgebildet ist, der mit den Dosierkavitäten (10) durch die Filterelemente (15) hindurch druckübertragend verbunden ist.

2. Dosiereinrichtung nach Anspruch 1,
**dadurch gekennzeichnet, dass** das Dosierorgan (8) um eine Rotationsachse (17) schwenkbar ist, wobei das Dosierorgan (8) in einer 0°-Rotationsposition mit seiner ebenen Transferfläche (9) nach oben weisend unterhalb der Dosierstation (5) liegt, und wobei das Dosierorgan (8) in einer 180°-Rotationsposition mit seiner ebenen Transferfläche (9) nach unten weisend oberhalb der Dosierstation (5) liegt.

3. Dosiereinrichtung nach Anspruch 2,
**dadurch gekennzeichnet, dass** mehrere Dosierorgane (8) um die Rotationsachse (17) herum angeordnet und für ein sequentielles Anfahren der Dosierstation (5) und der Füllstation (7) zu einer gemeinsam rotierbaren Einheit zusammengefasst sind.

4. Dosiereinrichtung nach einem der Ansprüche 1 bis 3,
**dadurch gekennzeichnet, dass** das Dosierorgan (8) in Richtung einer Hubachse (18) linear gegen die ebene Anlagefläche (11) der Dosierstation (5) verfahrbar und von ihr abhebbar ist.

5. Dosiereinrichtung nach einem der Ansprüche 1 bis 4,
**dadurch gekennzeichnet, dass** die ebene Transferfläche (9) des Dosierorgans (8) zumindest abschnittsweise durch ein elastisch nachgiebiges, geschlossen um die Dosierkavitäten (10) umlaufendes Dichtmaterial (19) gebildet ist.

6. Dosiereinrichtung nach Anspruch 5,
**dadurch gekennzeichnet, dass** das Dichtmaterial (19) luftdicht ist.

7. Dosiereinrichtung nach Anspruch 5 oder 6,
**dadurch gekennzeichnet, dass** das Dichtmaterial (19) luftdurchlässig porös ist.

8. Dosiereinrichtung nach einem der Ansprüche 5 bis 7,
**dadurch gekennzeichnet, dass** die Dosierkavitäten (10) einen unnachgiebigen, umlaufenden Rand (20) aufweisen, wobei der Rand (20) zur direkten Anlage an der Anlagefläche (11) der Dosierstation (5) vorgesehen ist, und wobei das Dichtmaterial (19) um den Rand (20) herumläuft.

9. Dosiersystem, umfassend die Dosiereinrichtung (1) nach einem der Ansprüche 1 bis 8 sowie eine zugeordnete Anzahl von in einer gemeinsamen Ebene (4) angeordneten Zielkavitäten (3).

10. Verfahren zur Dosierung von insbesondere pharmazeutischem Pulver mittels einer Dosiereinrichtung (1) nach einem der Ansprüche 1 bis 9 und zur gleichzeitigen Befüllung von mehreren in einer gemeinsamen Ebene (4) angeordneten Zielkavitäten (3), umfassend folgende Verfahrensschritte:
- Das Dosierorgan (8) wird mit seiner ebenen Transferfläche (9) dichtend an die ebene Anlagefläche (11) der Dosierstation (5) angelegt;
- durch den zentralen Druckkanal (16) wird durch die Filterelemente (15), die Dosierkavitäten (10) und die Pulverkanäle (13) hindurch eine negative Druckdifferenz auf das Pulver (2) im Pulvervorratsbehälter (6) aufgebracht, in dessen Folge sich die Dosierkavitäten (10) durch die ihnen zugeordneten Pulverkanäle (13) aus dem Pulvervorratsbehälter (6) gleichzeitig mit dem Pulver (2) befüllen, wobei das Pulver (2) an den Filterelementen (15) zurückgehalten wird;
- unter Aufrechterhaltung einer negativen Druckdifferenz wird das Dosierorgan (8) von der Dosierstation (5) zur Füllstation (7) verfahren, wobei die Dosierkavitäten (10) in Überdeckung mit zugeordneten Zielkavitäten (3) gebracht werden;
- die negative Druckdifferenz wird aufgehoben und das Pulver (2) aus den Dosierkavitäten (10) in die jeweils zugeordneten Zielkavitäten (3) gebracht;
- das Dosierorgan (8) wird zurück zur Dosierstation (5) verfahren.

11. Verfahren nach Anspruch 10,
**gekennzeichnet durch** folgende Verfahrensschritte:
- Das Dosierorgan (8) wird in Richtung der Hubachse (18) linear gegen die ebene Anlagefläche (11) der Dosierstation (5) verfahren;
- **durch** Beaufschlagung mit der negativen Druckdifferenz werden die Dosierkavitäten (10) mit dem Pulver (2) befüllt;
- das Dosierorgan (8) wird in Richtung der Hubachse (18) linear von der ebenen Anlagefläche (11) der Dosierstation (5) abgehoben, wobei die negative Druckdifferenz aufgehoben wird;
- unter erneuter Aufbringung und Aufrechterhaltung einer negativen Druckdifferenz wird das Dosierorgan (8) von der Dosierstation (5) zur Füllstation (7) verfahren.

12. Verfahren nach Anspruch 10 oder 11,
**dadurch gekennzeichnet, dass** an der Dosierstation (5) für die Befüllung der Dosierkavitäten (10) mit dem Pulver (2) eine impulsartige negative Druckdifferenz aufgebracht wird.

13. Verfahren nach einem der Ansprüche 10 bis 12,
**dadurch gekennzeichnet, dass** an der Füllstation (7) für die Überführung des Pulvers (2) aus den Dosierkavitäten (10) in die Zielkavitäten (3) ein Druckstoß mit positiver Druckdifferenz, ein zwischen positiver und negativer Druckdifferenz oszillierender Luftdruck, ein mechanischer Impuls oder eine mechanische Schwingung auf das in den Dosierkavitäten (10) befindliche Pulver (2) aufgebracht wird.

14. Verfahren nach einem der Ansprüche 10 bis 13,
**dadurch gekennzeichnet, dass** an der Füllstation (7) das Dosierorgan (8) mit seiner ebenen Transferfläche (9) dichtend an Siegelflächen (21) der Zielkavitäten (3) angedrückt wird.

## Claims

1. Metering device (1) for the volumetric metering of an in particular pharmaceutical powder (2) and for the simultaneous filling of a plurality of target cavities (3) with the metered powder (2), the plurality of target cavities (3) being arranged in a common plane (4), the device (1) comprising a metering station (5) with a powder reservoir (6),
**characterised in that** the metering device (1) further comprises a filling station (7) and a movable metering element (8), wherein the metering element (8) can be moved cyclically from the metering station (5) to the filling station (7) and back, wherein the metering element (8) has a flat transfer surface (9), into which a plurality of metering cavities (10) corresponding to the number and distribution of the target cavities (3) is incorporated, wherein the metering station (5) has a flat contact surface (11) provided for the sealing contact of the metering element (8) by means of its transfer surface (9), wherein metering openings (12) corresponding to the number and distribution of the metering cavities (10) and terminating into powder passages (13) starting from the powder reservoir (6) are provided in the contact surface (11), wherein each of the metering cavities (10) has in the plane of the transfer surface (9) an open side (14) and, opposite thereto, a base, wherein at least a section of the base of the respective metering cavity (10) is represented by an air-permeable filter element (15), wherein a central pressure passage (16) connected to the metering cavities (10) through the filter elements (15) is formed in the metering element (8) for the transmission of pressure.

2. Metering device according to claim 1,
**characterised in that** the metering element (8) is pivotable about an axis of rotation (17), the metering element (8) lying below the metering station (5) with its flat transfer surface (9) pointing upwards in a 0° rotational position and the metering element (8) lying above the metering station (5) with its flat transfer surface (9) pointing downwards in a 180° rotational position.

3. Metering device according to claim 2,
**characterised in that** a plurality of metering elements (8) is arranged around the axis of rotation (17) and combined to form a jointly rotating unit for a sequential start of the metering station (5) and the filling station (7).

4. Metering unit according to any of claims 1 to 3,
**characterised in that** the metering element (8) can be moved in the direction of a stroke axis (18) in a linear fashion against the flat contact surface (11) of the metering station (5) and lifted off therefrom.

5. Metering unit according to any of claims 1 to 4,
**characterised in that** at least a section of the flat transfer surface (9) of the metering element (8) is represented by an elastically yielding sealing material (19), which continuously surrounds the metering cavities (10).

6. Metering device according to claim 5,
**characterised in that** the sealing material (19) is airtight.

7. Metering device according to claim 5 or 6,
**characterised in that** the sealing material (19) is air-permeably porous.

8. Metering unit according to any of claims 5 to 7,
**characterised in that** the metering cavities (10) have an unyielding continuous edge (20), the edge (20) being provided for direct contact with the contact surface (11) of the metering station (5) and the sealing material (19) running around the edge (20).

9. Metering system, comprising the metering device (1) according to any of claims 1 to 8 and an assigned number of target cavities (3) arranged in a common plane (4).

10. Method for the metering an in particular pharmaceutical powder by means of a metering device (1) according to any of claims 1 to 9 and for the simultaneous filling of a plurality of target cavities (3) arranged in a common plane (3), the method comprising the following steps:
- the metering element (8) is placed with its flat transfer surface (9) against the flat contact surface (11) of the metering station (5) while forming a seal;
- a negative pressure differential is applied to the powder (2) in the powder reservoir (6) through the central pressure passage (16) via the filter elements (15), the metering cavities (10) and the powder passages (13), resulting in the simultaneous filling of the metering cavities (10) with the powder (2) from the powder reservoir (6) through the associated powder passages (13), the powder (2) being held back at the filter elements (15);
- while a negative pressure differential is maintained, the metering element (8) is moved from the metering station (5) to the filling station (7), the metering cavities (10) being superimposed on the associated target cavities (3);
- the negative pressure differential is cancelled and the powder (2) is moved from the metering cavities (10) into the respective associated target cavities (3);
- the metering element (8) is moved back to the metering station (5).

11. Method according to claim 10,
**characterised by** the following steps:
- the metering element (8) is moved in the direction of the stroke axis (18) in a linear fashion against the flat contact surface (11) of the metering station (5);
- the metering cavities (10) are filled with the powder (2) by the application of the negative pressure differential;
- the metering element (8) is lifted off the flat contact surface (11) of the metering station (5) in the direction of the stroke axis (18), the negative pressure differential being cancelled;
- while a negative pressure differential is once again applied and maintained, the metering element (8) is moved from the metering station (5) to the filling station (7).

12. Method according to claim 10 or 11,
**characterised in that** a pulsed negative pressure differential is applied to the metering station (5) for filling the metering cavities (10) with the powder (2).

13. Method according to any of claims 10 to 12,
**characterised in that** a pressure surge with a positive pressure differential, an air pressure oscillating between a positive and a negative pressure differential, a mechanical impulse or a mechanical vibration is applied to the powder (2) in the metering cavities (10) for transferring the powder (2) from the metering cavities (10) to the target cavities (3).

14. Method according to any of claims 10 to 13,
**characterised in that** at the filling station (7) the metering element (8) is pushed with its flat transfer surface (9) against sealing surfaces (21) of the target cavities (3) while forming a seal.

## Revendications

1. Dispositif de dosage (1) pour le dosage volumétrique d'une poudre (2) en particulier pharmaceutique (2) et pour le remplissage simultané de plusieurs cavités cibles (3) avec la poudre (2) dosée, étant précisé que lesdites cavités cibles (3) sont disposées dans un plan commun (4), comprenant une station de dosage (5) avec un réservoir de poudre (6),
**caractérisé en ce que** le dispositif de dosage (1) comprend par ailleurs une station de remplissage (7) et un organe de dosage mobile (8), que l'organe de dosage (8) est mobile cycliquement de la station de dosage (5) jusqu'à la station de remplissage (7) et dans l'autre sens, que l'organe de dosage (8) comporte une surface de transfert plane (9) dans laquelle sont réalisées un nombre de cavités de dosage (10) correspondant au nombre et à la répartition géométrique des cavités cibles (3), que la station de dosage (5) comporte une surface d'application plane (11) qui est destinée à une application étanche de l'organe de dosage (8) à l'aide de la surface de transfert (9) de celui-ci, qu'un nombre d'ouvertures de dosage (12) correspondant au nombre et à la répartition géométrique des cavités de dosage (10) sont formées dans la surface d'application (11), dans lesquelles débouchent les conduits de poudre (13) qui partent du réservoir de poudre (6), que les cavités de dosage (10) présentent chacune dans le plan de la surface de transfert (9) un côté ouvert (14) et, en face, un fond, que le fond de chaque cavité de dosage (10) est formé au moins par zones par un élément filtrant perméable à l'air (15), qu'il est prévu, formé dans l'organe de dosage (8), un conduit de pression central (16) qui est relié avec une transmission de pression à travers les éléments filtrants (15) aux cavités de dosage (10).

2. Dispositif de dosage selon la revendication 1,
**caractérisé en ce que** l'organe de dosage (8) est apte à pivoter sur un axe de rotation (17), étant précisé que l'organe de dosage (8), dans une position de rotation à 0°, est disposé avec sa surface de transfert plane (9) dirigée vers le haut, au-dessous de la station de dosage (5), et que l'organe de dosage (8), dans une position de rotation à 180°, est disposé avec sa surface de transfert plane (9) dirigée vers le bas, au-dessus de la station de dosage (5).

3. Dispositif de dosage selon la revendication 2,
**caractérisé en ce que** plusieurs organes de dosage (8) sont disposés autour de l'axe de rotation (17) et sont réunis en une unité apte à tourner conjointement, pour un rapprochement séquentiel de la station de dosage (5) et de la station de remplissage (7).

4. Dispositif de dosage selon l'une des revendications 1 à 3,
**caractérisé en ce que** l'organe de dosage (8) est mobile linéairement dans le sens d'un axe de course (18) pour être amené contre la surface d'application plane (11) de la station de dosage (5) et pour être détaché de ladite surface d'application (11).

5. Dispositif de dosage selon l'une des revendications 1 à 4,
**caractérisé en ce que** la surface de transfert plane (9) de l'organe de dosage (8) est formée au moins par zones par un matériau d'étanchéité (19) qui est flexible élastiquement, qui est fermé sur lui-même sur tout le tour des cavités de dosage (10).

6. Dispositif de dosage selon la revendication 5,
**caractérisé en ce que** le matériau d'étanchéité est étanche à l'air.

7. Dispositif de dosage selon la revendication 5 ou 6,
**caractérisé en ce que** le matériau d'étanchéité (19) est poreux et perméable à l'air.

8. Dispositif de dosage selon l'une des revendications 5 à 7,
**caractérisé en ce que** les cavités de dosage (10) comportent un bord circulaire inflexible (20), étant précisé que ce bord (20) est destiné à s'appliquer directement contre la surface d'application (11) de la station de dosage (5), et que le matériau d'étanchéité (19) fait tout le tour du bord (20).

9. Système de dosage comprenant le dispositif de dosage (1) selon l'une des revendications 1 à 8, ainsi qu'un nombre correspondant de cavités cibles (3) disposées dans un plan commun (4).

10. Procédé pour le dosage de poudre en particulier pharmaceutique à l'aide d'un dispositif de dosage (1) selon l'une des revendications 1 à 9 et pour le remplissage simultané de plusieurs cavités cibles (3) disposées dans un plan commun (4), comprenant les étapes de procédé suivantes :
- l'organe de dosage (8) est appliqué de manière étanche avec sa surface de transfert plane (9) contre la surface d'application plane (11) de la station de dosage (5) ;
- par le conduit de pression central (16), une différence de pression négative est appliquée à la poudre (2) dans le réservoir de poudre (6), à travers les éléments filtrants (15), les cavités de dosage (10) et les conduits de poudre (13), à la suite de quoi les cavités de dosage (10) se remplissent en même temps de poudre (2), à partir du réservoir de poudre (6) et par les conduits de poudre (13) qui leur sont associés, étant précisé que la poudre (2) est retenue au niveau des éléments filtrants (15) ;
- avec un maintien d'une différence de pression négative, l'organe de dosage (8) est déplacé de la station de dosage (5) jusqu'à la station de remplissage (7), étant précisé que les cavités de dosage (10) viennent couvrir les cavités cibles (3) associées ;
- la différence de pression négative est supprimée et la poudre (2) est amenée des cavités de dosage (10) dans les cavités cibles (3) qui leur sont associées respectivement ;
- l'organe de dosage (8) est ramené à la station de dosage (5).

11. Procédé selon la revendication 10,
**caractérisé par** les étapes de procédé suivantes :
- l'organe de dosage (8) est déplacé linéairement dans le sens d'un axe de course (18) pour être amené contre la surface d'application plane (11) de la station de dosage (5) ;
- grâce à l'application de la différence de pression, les cavités de dosage (10) sont remplies avec la poudre (2) ;
- l'organe de dosage (8) est détaché linéairement, dans le sens de l'axe de course (18), de la surface d'application plane (11) de la station de dosage (5), étant précisé que la différence de pression négative est supprimée ;
- avec une nouvelle application et un maintien d'une différence de pression négative, l'organe de dosage (8) est déplacé de la station de dosage (5) jusqu'à la station de remplissage (7).

12. Procédé selon la revendication 10 ou 11,
**caractérisé en ce qu'**au niveau de la station de dosage (5), une différence de pression négative par impulsions est appliquée pour le remplissage des cavités de dosage (10) avec la poudre (2).

13. Procédé selon l'une des revendications 10 à 12,
**caractérisé en ce qu'**au niveau de la station de remplissage (7), pour le passage de la poudre (2) des cavités de dosage (10) dans les cavités cibles (3) un choc de pression avec une différence de pression positive, une pression d'air oscillant entre une différence de pression positive et négative, une impulsion mécanique ou une vibration mécanique est appliqué à la poudre (2) qui se trouve dans les cavités de dosage (10).

14. Procédé selon l'une des revendications 10 à 13,
**caractérisé en ce qu'**au niveau de la station de remplissage (7), l'organe de dosage (8) est pressé de manière étanche, avec sa surface de transfert plane (9), contre des surfaces de scellement (21) des cavités cibles (3).
